# DEMANDE DE BREVET EUROPEEN

(11) **EP 0 679 374 A1**
(43) Date de publication de la demande: **02.11.1995**
(21) Numéro de dépôt: 95400931.2
(22) Date de dépôt: 25.04.1995
(51) Int. Cl.: A61F 2/06

(54) **Endoprothèse pour le traitement d'un vaisseau sanguin**

(30) Priorité: 26.04.1994 FR 9405034
(71) Demandeur: LABORATOIRE PEROUSE IMPLANT, F-60540 Bornel (FR)
(72) Inventeur: Perouse, Eric, F-95290 L'Isle Adam (FR)
(74) Mandataire: Polus, Camille

(57) **Abrégé**

Ce dispositif de traitement d'un vaisseau sanguin comprend une gaine extérieure (10) contenant d'une part une endoprothèse (1) dont l'extrémité distale est munie d'un lien (4), et d'autre part un tuteur (9), l'extrémité distale de l'endoprothèse (1) étant reliée au tuteur (9) de manière séparable par ledit lien (4).

Application au traitement de l'athérome artériel.

## Description

La présente invention est relative à un dispositif de traitement d'un vaisseau sanguin. Il s'applique en particulier au maintien des artères athéromateuses après dilatation, ou également au pontage des anévrismes.

L'invention a pour but de fournir un dispositif de traitement qui permette une mise en place précise de l'endoprothèse dans une artère sans risques d'endommager cette endoprothèse, et ce par des moyens simples, fiables et peu coûteux.

A cet effet, l'invention a pour objet un dispositif de traitement d'un vaisseau sanguin, caractérisé en ce qu'il comprend une gaine extérieure contenant d'une part une endoprothèse dont l'extrémité distale est munie d'un lien, et d'autre part un tuteur, l'extrémité distale de l'endoprothèse étant reliée au tuteur de manière séparable par ledit lien.

Le dispositif de traitement d'un vaisseau sanguin suivant l'invention peut comporter une ou plusieurs des caractéristiques suivantes :
- le tuteur est creux et au moins un brin du lien s'étend à travers le tuteur ;
- le lien comprend un fil de ligature formant boucle comportant deux brins enfilés librement autour de ladite extrémité distale et se prolongeant à distance de l'endoprothèse ;
- le lien comprend un fil de ligature formant boucle fixé en un point à l'endoprothèse et comportant au moins un brin qui s'étend à distance de l'endoprothèse ;
- le lien comporte un fil de ligature formant un noeud coulant et se terminant par un organe d'accrochage ;
- le tuteur porte un ballonnet gonflable entouré par l'endoprothèse, laquelle est d'un type plastiquement déformable ;
- l'endoprothèse est auto-expansible ; et
- l'endoprothèse comporte à son extrémité proximale un second lien formant un lien d'étranglement sélectif.

Des exemples de réalisation de l'invention vont maintenant être décrits en regard des dessins annexés, sur lesquels :
- la figure 1 est une vue de côté avec arrachement d'une endoprothèse implantée dans une artère fémorale d'une jambe ;
- la figure 2 représente les moyens de récupération de cette endoprothèse ;
- les figures 3 et 4 illustrent les phases de la récupération de l'endoprothèse implantée ;
- la figure 5 montre une variante de l'endoprothèse de la figure 1 ;
- la figure 6 est une vue de côté d'un dispositif de traitement d'un vaisseau sanguin suivant l'invention par mise en place d'une endoprothèse ;
- les figures 7 à 9 illustrent les étapes de la mise en place de l'endoprothèse de la figure 6 ; et
- la figure 10 est une vue de côté d'un dispositif de traitement par mise en place d'une autre variante d'endoprothèse suivant l'invention.

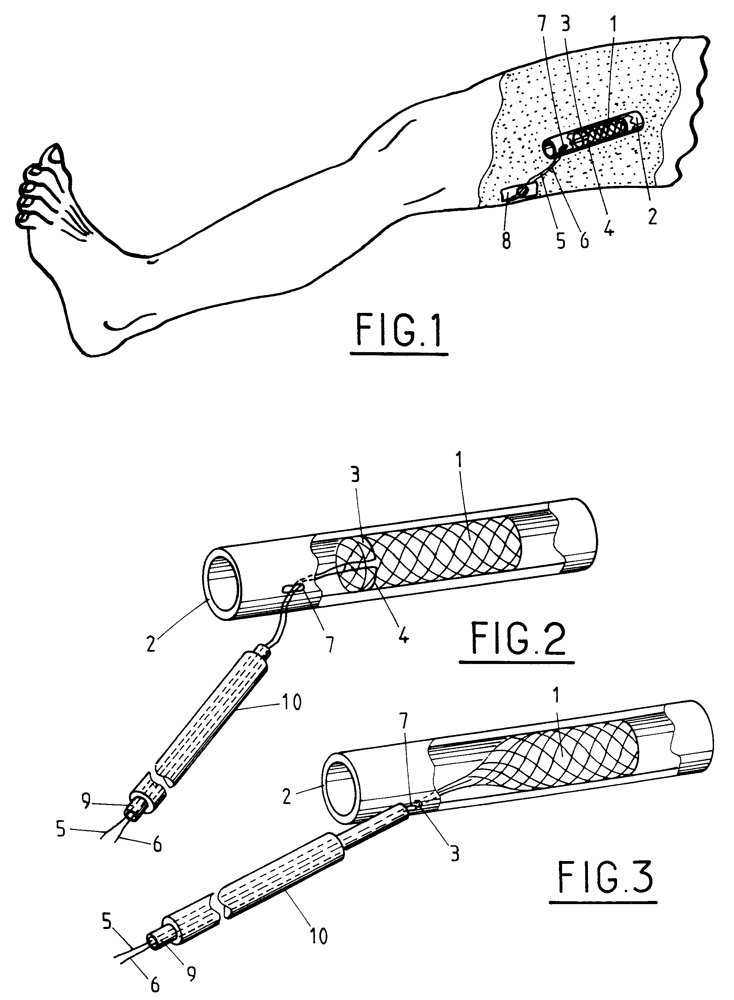

On voit à la figure 1 une endoprothèse tubulaire 1 implantée dans l'artère fémorale 2 d'un patient. Cette endoprothèse est pour l'essentiel d'un type connu, décrit dans la demande de brevet français FR-A-2 688 401. Cette endoprothèse 1 est constituée d'un treillis tubulaire noyé dans un film. Le treillis est constitué d'acier inoxydable de qualité biocompatible. Le film est en matière extensible et étanche aux liquides, telle qu'un élastomère. L'endoprothèse 1 est par exemple auto-expansible en utilisant un acier inoxydable ayant des propriétés de ressort.

L'endoprothèse 1 auto-expansible peut être mise en place par un outil de mise en place approprié, par exemple celui décrit dans la demande de brevet français FR-A-2 688 688, dans lequel l'endoprothèse est comprimée radialement dans une tulipe d'extrémité de l'outil. La tulipe est découpée in situ en pétales pour libérer l'endoprothèse.

La figure 1 de la présente demande montre que l'une des extrémités 3 de l'endoprothèse tubulaire 1 porte autour de sa circonférence un fil de ligature 4 dont les deux brins 5 et 6 traversent la paroi de l'artère 2 par une ouverture 7 puis traversent la peau pour être lovés et fixés à la surface de la peau du patient par un pansement 8.

Le fil de ligature 4 est enfilé et entrelacé librement, c'est-à-dire avec possibilité de coulissement, dans les mailles de l'extrémité 3 de l'endoprothèse 1 comme le montre la figure 2, et ses deux brins en ressortent l'un à côté de l'autre, à travers la même maille du treillis et au voisinage de l'intersection proximale de cette maille.

La récupération de l'endoprothèse 1 va maintenant être décrite en regard des figures 2 à 4.

On voit sur la figure 2 que, pour récupérer l'endoprothèse, les brins 5 et 6 de fil sont déroulés et passés dans un cathéter ou tuteur 9 lui-même emmanché dans une gaine 10, de longueur inférieure. Ce cathéter 9 et cette gaine 10 sont dans un premier temps situés à l'extérieur du patient.

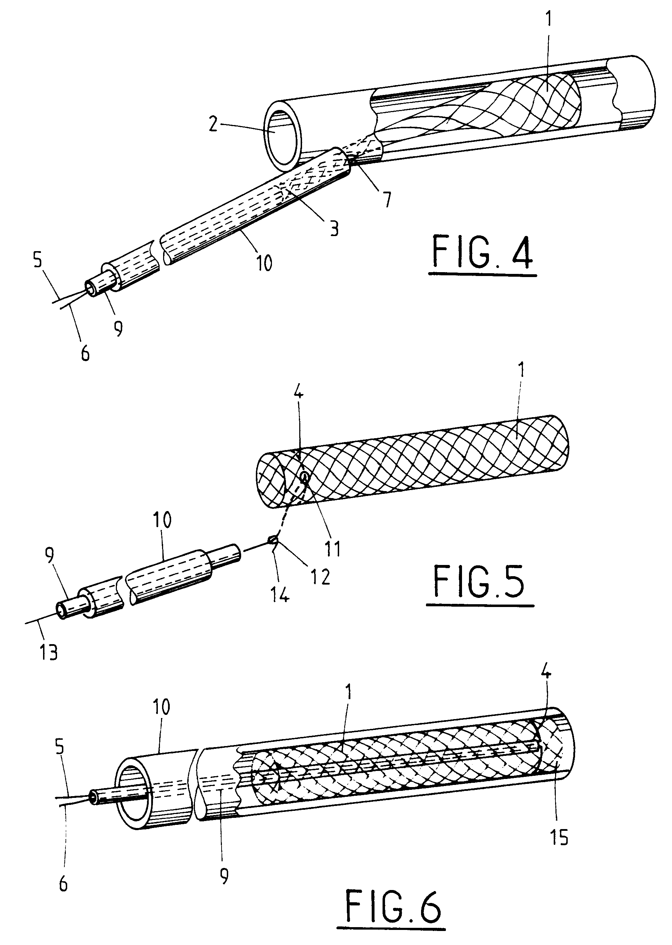

Le cathéter 9 est ensuite glissé sur les brins 5 et 6 (figure 3), il traverse la peau et l'artère du patient jusqu'à atteindre l'extrémité 3 de l'endoprothèse. Les brins 5 et 6 sont alors tendus par l'opérateur, ce qui entraîne un étranglement de cette extrémité 3. En maintenant les brins 5 et 6 fixés tendus et le cathéter 9 immobile, l'opérateur fait coulisser la gaine 10 jusqu'à l'extrémité étranglée de l'endoprothèse, par l'ouverture 7. L'extrémité distale de la gaine 10 avance alors sur l'extrémité proximale 3 de l'endoprothèse (figure 4), qui reste immobile dans l'artère pendant toute l'opération. Puis la gaine pénètre à l'intérieur de l'artère 2 jusqu'à ce qu'elle enveloppe ou "avale" l'endoprothèse 1 sur toute sa longueur. Cette dernière est alors complètement séparée de la paroi de l'artère.

L'ensemble brins tendus, cathéter et gaine enveloppant l'endoprothèse comprimée radialement peut ensuite être retiré doucement du patient ; l'endoprothèse 1 est ainsi récupérée.

En variante, le fil 4 pourrait être fixé par une extrémité à l'extrémité proximale de l'endoprothèse, faire le tour de celle-ci en s'entrelaçant dans le treillis, et repartir librement en un seul brin.

La figure 5 illustre une variante du dispositif d'accrochage. Le fil de ligature 4 fait le tour de l'extrémité proximale de l'endoprothèse 1, une de ses extrémités formant un oeillet de noeud coulant 11 traversé par l'autre extrémité du fil, qui porte un anneau d'accrochage 12. Lorsque l'on veut comprimer cette extrémité, il suffit, par angioscopie, d'accrocher à l'anneau 12 un fil auxiliaire 13 pourvu à son extrémité distale d'un crochet 14 et sur lequel est ensuite enfilé le cathéter 9 comme précédemment décrit. La récupération de l'endoprothèse est ensuite réalisée de la même manière que précédemment, au moyen du cathéter 9 et de la gaine 10.

La mise en place de l'endoprothèse auto-expansible 1 dans l'artère 2 est réalisée suivant l'invention par le dispositif illustré à la figure 6. L'endoprothèse 1 est maintenant munie à son extrémité distale 15, du fil 4 de ligature passé à travers les mailles de la circonférence, en se terminant par les brins 5 et 6, de la même manière qu'à la figure 2.

Le dispositif de traitement comprend les mêmes éléments que précédemment, à savoir un cathéter 9 et une gaine 10. Les brins 5 et 6 sont passés à l'intérieur du cathéter 9, qui forme tuteur et est lui-même disposé à l'intérieur de l'endoprothèse 1 sur toute la longueur de celle-ci. L'ensemble endoprothèse comprimée et cathéter est inséré dans la gaine 10.

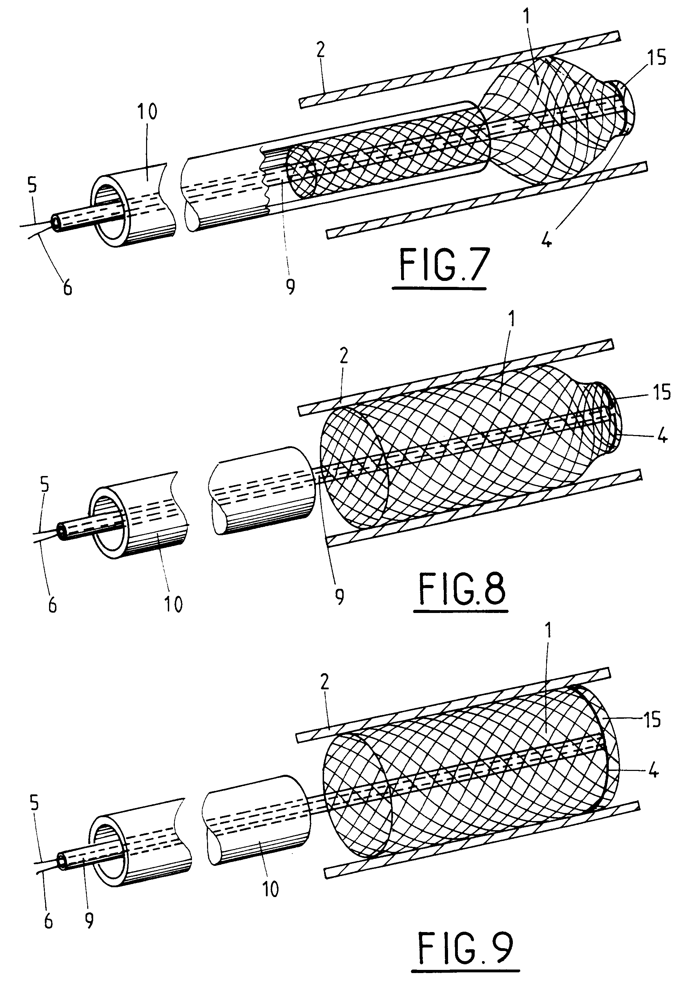

Pour faciliter la pénétration de ce dispositif jusqu'à l'artère, un guide filiforme peut s'étendre de l'extérieur du patient jusqu'à l'emplacement de l'artère où doit être posée l'endoprothèse 1. Lorsque l'endoprothèse est correctement disposée, le cathéter est maintenu en place et les brins 5,6 sont légèrement tendus, pendant que l'on fait glisser la gaine 10 vers l'arrière, comme le montrent les figures 7 à 9. Pendant cette opération, l'endoprothèse est maintenue immobile par son extrémité distale 15 par le cathéter 9 et les brins 5,6, et sa partie située entre le fil 4 et l'extrémité distale de la gaine 10 se dilate et s'applique contre la paroi de l'artère.

Lorsque l'endoprothèse a été totalement libérée de la gaine, les brins 5 et 6 sont relâchés (figure 9). L'opérateur tire sur l'un des deux brins pour retirer totalement le fil 4 du patient, le cathéter et la gaine sont également enlevés. L'endoprothèse est alors en position dans l'artère 2.

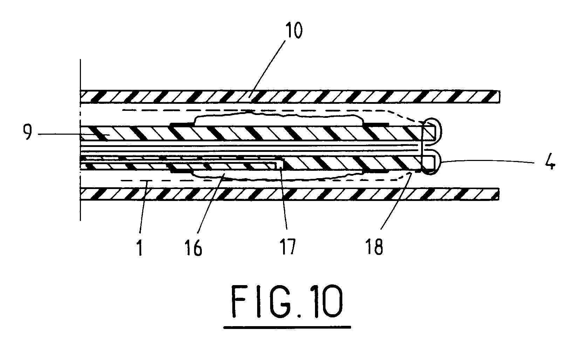

Dans le cas où l'endoprothèse 1 n'est pas auto-expansible mais est réalisée à partir d'un treillis en matériau plastiquement déformable, sa dilatation est assurée par un ballonnet. Le dispositif de traitement d'une telle endoprothèse peut être constitué (figure 10) d'une gaine 10 enveloppant un cathéter 9 sur lequel est fixé par ses deux extrémités un ballonnet 16 à expansion radiale. Un conduit 17 d'amenée de fluide d'expansion, prévu dans la paroi du cathéter, débouche radialement vers l'extérieur entre les deux extrémités du ballonnet 16. L'endoprothèse 1, à l'état rétracté radialement, est disposée par-dessus le ballonnet 16, entre le cathéter 9 et la gaine 10. L'extrémité distale 18 de l'endoprothèse 1 est fixée à l'extrémité distale du cathéter 9 par le fil 4, qui forme comme précédemment une boucle entrelacée dans le treillis de l'endoprothèse et s'étendant sur la circonférence de celle-ci, et dont les deux brins 5 et 6 sont enfilés vers l'arrière dans le cathéter.

Lorsque ce dispositif est introduit jusqu'à l'emplacement désiré, la gaine est retirée en maintenant le cathéter immobile. Les brins 5 et 6 sont détendus, puis le ballonnet 16 est gonflé pour dilater l'endoprothèse 1 en métal malléable, puis est dégonflé. Le cathéter 9 est alors extrait doucement en laissant l'endoprothèse en place, et le fil 4 est finalement retiré par traction sur l'un des deux brins 5 et 6.

Comme on le comprend, l'endoprothèse peut être munie d'un fil 4 à chaque extrémité, afin de permettre à la fois de la mettre en place sans pousser sur son extrémité proximale et de la récupérer facilement.

Enfin, le dispositif suivant l'invention a pour avantage de rendre possible la correction d'une erreur de positionnement de l'endoprothèse dans le vaisseau, en l'étranglant à nouveau à l'aide du fil afin de la déplacer à l'emplacement désiré.

## Revendications

**1 -** Dispositif de traitement d'un vaisseau sanguin, caractérisé en ce qu'il comprend une gaine extérieure (10) contenant d'une part une endoprothèse (1) dont l'extrémité distale est munie d'un lien (4), et d'autre part un tuteur (9), l'extrémité distale de l'endoprothèse (1) étant reliée au tuteur (9) de manière séparable par ledit lien (4).

**2 -** Dispositif selon la revendication 1, caractérisé en ce que le tuteur (9) est creux et en ce qu'au moins un brin du lien (4) s'étend à travers le tuteur (9).

**3 -** Dispositif selon la revendication 1 ou 2, caractérisé en ce que le lien (4) comprend un fil de ligature formant boucle comportant deux brins (5, 6) enfilés librement autour de ladite extrémité distale et se prolongeant à distance de l'endoprothèse.

**4 -** Dispositif selon la revendication 1 ou 2, caractérisé en ce que le lien comprend un fil de ligature formant boucle fixé en un point à l'endoprothèse et comportant au moins un brin qui s'étend à distance de l'endoprothèse.

**5 -** Dispositif selon la revendication 1 ou 2, caractérisé en ce que le lien (4) comporte un fil de ligature formant un noeud coulant (11) et se terminant par un organe d'accrochage (12).

**6 -** Dispositif selon l'une quelconque des revendications 1 à 5, caractérisé en ce que le tuteur (9) porte un ballonnet gonflable (16) entouré par l'endoprothèse (1), laquelle est d'un type plastiquement déformable.

**7 -** Dispositif selon l'une quelconque des revendications 1 à 5, caractérisé en ce que l'endoprothèse (1) est auto-expansible.

**8.** Dispositif selon la revendication 7, caractérisé en ce que l'endoprothèse (1) comporte à son extrémité proximale un second lien formant un lien d'étranglement sélectif.
